# EUROPÄISCHE PATENTSCHRIFT

(11) **EP 0 533 000 B1**
(45) Veröffentlichungstag und Bekanntmachung des Hinweises auf die Patenterteilung: **02.07.1997**
(21) Anmeldenummer: 92115206.2
(22) Anmeldetag: 05.09.1992
(51) Int. Cl.: C07D 217/26

(54) **Verfahren zur Herstellung von 1,2,3,4-Tetrahydro-3-isochinolin-Derivaten**
Process for the preparation of 3-substituted 1,2,3,4-tetrahydro isoquinoline derivatives
Procédé de préparation de 1,2,3,4-tétrahydro-isoquinoléines substituées en 3

(30) Priorität: 18.09.1991 CH 2752/91; 06.07.1992 CH 2130/92
(43) Veröffentlichungstag der Anmeldung: 24.03.1993
(73) Patentinhaber: F.HOFFMANN-LA ROCHE & CO. AKTIENGESELLSCHAFT, CH-4002 Basel (CH)
(72) Erfinder: Gokhale, Surendra, CH-4058 Basel (CH); Schlageter, Markus, CH-4103 Bottmingen (CH)
(74) Vertreter: Kellenberger, Marcus, Dr.

(56) Entgegenhaltungen:
- US-A- 4 912 221
- BERICHTE DER DEUTSCHEN CHEMISCHEN GESELLSCHAFT Bd. 44, 10. Juli 1911, BERLIN(DE), Seiten 2030 - 2036 A. PICTET UND T. SPENGLER 'Uber die Bildung von Isochinolin-derivaten durch Einwirkung von Methylal auf Phenyl-athylamin, Phenyl-alanin und Tyrosin'
- DATABASE WPIL, Week 9036, Derwent Publications Ltd., London, GB; AN 90-272653

## Beschreibung

Die vorliegende Erfindung betrifft ein neues Verfahren zur Herstellung von 1,2,3,4-Tetrahydro-3-isochinolin-Derivaten der allgemeinen Formel 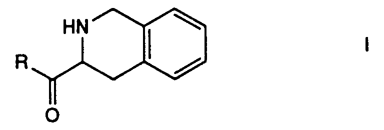 worin R Hydroxy, nieder Alkylamino, nieder Alkoxy oder Phenyl-C₂₋₆-Alkoxy, welches am Phenylring durch nieder Alkyl oder nieder Alkoxy ein- oder mehrfach substituiert sein kann, bedeutet.

Der in dieser Beschreibung verwendete Ausdruck "nieder Alkyl" - allein oder in Kombination - betrifft geradkettige und verzweigte Alkylgruppen mit 1-6, vorzugsweise 1-4, Kohlenstoffatomen, wie Methyl, Aethyl, Propyl, Isopropyl, Butyl, Isobutyl, sec.-Butyl, tert.-Butyl, Pentyl, Hexyl und dergleichen. Der Ausdruck "nieder Alkoxy" bedeutet nieder Alkyläthergruppen, worin der Ausdruck "nieder Alkyl" die obige Bedeutung hat, wie Methoxy, Aethoxy, Propoxy, Isopropoxy, Butoxy, Isobutoxy, sec.-Butoxy, tert.-Butoxy, Pentyloxy, Hexyloxy und dergleichen. In ähnlicher Weise bedeutet der Ausdruck "C₂₋₆-Alkoxy" Alkyläthergruppen mit 2-6 Kohlenstoffatomen wie oben definiert.

Bevorzugt ist die Herstellung solcher Verbindungen der Formel I, worin R nieder Alkylamino, vorzugsweise Isobutylamino oder tert.-Butylamino, besonders bevorzugt tert.-Butylamino, bedeutet.

Die Tetrahydroisochinolin-Derivate der Formel I lassen sich zu den entsprechenden Decahydroisochinolin-Derivaten der allgemeinen Formel 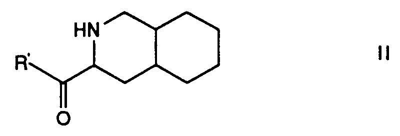 worin R' Hydroxy, nieder Alkylamino, nieder Alkoxy oder Cyclohexyl-C₂₋₆-Alkoxy, welches am Cyclohexylring durch nieder Alkyl oder nieder Alkoxy ein- oder mehrfach substituiert sein kann, bedeutet,
hydrieren. Diese Decahydroisochinolin-Derivate sind wertvolle Bausteine in der Synthese von Proteasehemmern wie sie in EP-A 0.346.847 und 0.432.695 beschrieben sind. Insbesondere eignet sich eines der Decahydroisochinolin-Derivate der Formel II, nämlich das N-t-Butyl-decahydro-(4aS,8aS)-isochinolin-3(S)-carboxamid zur Herstellung von den in EP-A 0.432.695 beschriebenen Proteasehemmern der allgemeinen Formel 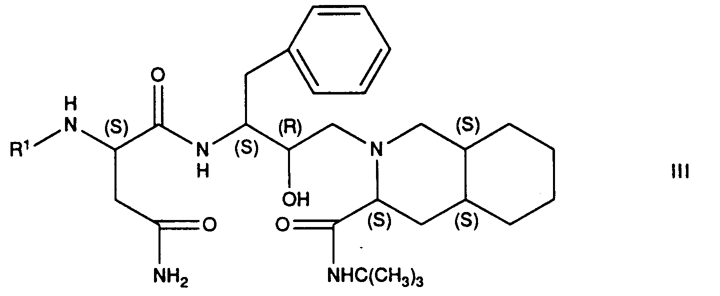 worin R¹ Benzyloxycarbonyl oder 2-Chinolylcarbonyl bedeutet.

Die Ueberführung der Decahydroisochinolin-Derivate der Formel II in die Proteasehemmer ist ebenfalls in den beiden oben genannten europäischen Patentpublikationen beschrieben.

Die Herstellung von Tetrahydroisochinolin-Derivaten nach Pictet-Spengler ausgehend von einem Phenethylamin-Derivat und Formaldehyd in Gegenwart von Salzsäure ist aus der Literatur bekannt und stellt bis heute praktisch das einzige gangbare Verfahren zur Herstellung dieser Substanzklasse dar [vgl. US-Patentschriffen 3.836.536 und 3.906.099 sowie Europäische Patentpublikationen Nos. 0.012.845 und 0.029.488, welche allesamt auf dieselbe Literaturstelle verweisen, nämlich J. Amer. Chem. Soc. 70, 182 (1948); vgl. auch Europäische Patentpublikationen Nos. 0.018.104, 0.039.804 und 0.104.546 sowie Heterocycles, 29, 6, (1989) 1179-1183], obwohl in der Fachwelt allgemein bekannt ist, dass bei der Pictet-Spengler-Reaktion durch die Wirkung der Salzsäure auf den Formaldehyd [Chem. Rev. 55 (1955), 314] die beiden äusserst carzinogenen α-Haloether Chlormethylmethylether (CMME) und Bis(chlormethyl)methylether (BCME) als Nebenprodukte gebildet werden. Die Carzinogenität dieser beiden α-Haloether, insbesondere diejenige von BCME, ist so gross, dass heute ein Verfahren, bei welchem diese Verbindungen als Nebenprodukte gebildet werden, aus Sicherheitsgründen für eine technische Verwertung nicht mehr in Frage kommt [Arch Environ Health, 30 (1975) 61-72].

Ein weiterer Nachteil der Pictet-Spengler-Synthese liegt darin, dass bei der Cyclisation unaktivierter chiraler Phenethylamin-Derivate aufgrund der rigorosen Reaktionsbedingungen mit partieller Racemisierung zu rechnen ist; so tritt gemäss Chem. Pharm. Bull. Japan 31, 312 (1983) im Falle des L-Phenylalanins zu etwa 32% Racemisierung auf.

Gemäss Chem. Pharm. Bull. Japan 25, 1732 (1977) lassen sich unaktivierte Systeme, wie 2-Phenethylamin, in Form von Acylderivaten (z.B. des Tosylderivats) unter sehr milden Reaktionsbedingungen mittels Formaldehyd in Gegenwart von Bortrifluorid cyclisieren. Eigene Versuche haben indessen gezeigt, dass sich zwar N-Tosyl-(L)-phenylanalylbenzylester mittels Formaldehyd in Gegenwart von Bortrifluorid ohne Racemisierung zum entsprechenden Tetrahydroisochinolinderivat cyclisieren liess, dass aber die Abspaltung der Tosylgruppe im Cyclisationsprodukt in präparativem Ausmass nicht gelang. Es gelang zwar, ein anderes Acylderivat von L-Phenylalanin, nämlich N-Benzyloxycarbonyl-L-phenylalanin-t-butylamid mittels Formaldehyd in Gegenwart von Bortrifluorid ohne Racemisierung zu Benzyl 3(S)-(t-butylcarbamoyl)-1,2,3,4-tetrahydro-3-isochinolincarboxylat zu cyclisieren und hiervon die Benzyloxygruppe hydrogenolytisch abzuspalten; da aber fur die Cyclisation Bortrifluorid, sei es als Komplex mit dem verfahrenstechnisch problematischen Diethylether oder als Komplex mit Essigsäure, in 1^{1/2}- bis 2-fachem Ueberschuss verwendet werden musste, ergaben sich dabei Probleme bezüglich der Entsorgung bzw. Rückgewinnung von Borverbindungen nach Durchführung der Cyclisation.

Im Rahmen der vorliegenden Erfindung wurde nun festgestellt, dass sich Phenethylamin-Derivate der allgemeinen Formel 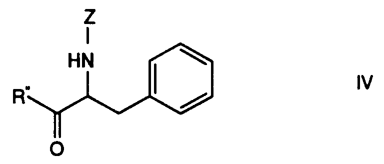 worin Z Benzyloxycarbonyl und R'' Hydroxy, nieder Alkylamino, nieder Alkoxy oder Phenyl-nieder Alkoxy, welches am Phenylring durch nieder Alkyl oder nieder Alkoxy ein- oder mehrfach substituiert sein kann, bedeuten,
in guter Ausbeute, ohne Racemisierung und ohne dass sich wesentliche Probleme bezüglich Verfahrenstechnik und Sicherheit stellen, mit Formaldehyd zu den entsprechenden Tetrahydroisochinolin-Derivaten der allgemeinen Formel 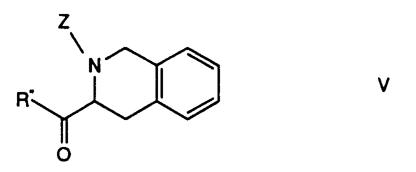 worin R'' und Z obige Bedeutung besitzen,
cyclisieren lassen, wenn man die Umsetzung in Gegenwart von Schwefelsäure in Essigsäure durchführt. Das erfindungsgemässe Verfahren ist dadurch gekennzeichnet, dass man ein Phenethylamin-Derivat der Formel IV bei einer Temperatur zwischen etwa Raumtemperatur und etwa 50°C in Gegenwart von Schwefelsäure in Essigsäure mit Formaldehyd umsetzt und in der erhaltenen Verbindung der Formel V die Gruppe Z abspaltet.

Anstelle von Formaldehyd bzw. einer wässrigen Formaldehydlösung kann auch Paraformaldehyd oder ein geeignetes Acetal des Formaldehyds verwendet werden, insbesondere ein Di-nieder-Alkylacetal, wie Dimethoxymethan. Die Reaktionstemperatur liegt zwischen etwa Raumtemperatur und 50°C bei Verwendung von Formaldehyd oder Paraformaldehyd, vorzugsweise zwischen 45° und 50°C. Bei Verwendung von Dimethoxymethan darf die Reaktionstemperatur 40°C nicht übersteigen (Siedepunkt von Dimethoxymethan: 41-42°C). Die Umsetzung erfolgt in einem unter den Reaktionsbedingungen inerten Lösungsmittel, wie Toluol, oder in einem Ueberschuss an Essigsäure, vorzugsweise in Toluol. Die Reaktionsdauer beträgt in Abhängigkeit von den genauen Reaktionsbedingnngen etwa 7 bis etwa 24 Stunden. Die besten Resultate werden erzielt, wenn das Phenethylamin-Derivat der Formel IV in Toluol mit 3 Mol-Aequivalenten Dimethoxymethan und 2 Mol-Aequivalenten 7,2 M Schwefelsäure in Essigsäure 24 bis 25 Stunden bei 40°C zur Reaktion gebracht wird.

Die Abspaltung der Benzyloxycarbonylgruppe (Z) aus dem Cyclisationsprodukt der Formel V erfolgt zweckmässigerweise hydrogenolytisch, z.B. durch Hydrierung in Gegenwart eines Palladiumkatalysators (wie 10%-ige Palladiumkohle) in einem unter den Reaktionsbedingnngen inerten organischen Lösungsmittel, z.B. in einem niederen Alkanol, wie Ethanol oder dergleichen, wobei, falls R'' Benzyloxy bedeutet, die Benzylgruppe ebenfalls hydrogenolytisch abgespalten und die entsprechende Verbindung der Formel I, worin R Hydroxy bedeutet, erhalten wird.

Die Reduktion der erfindungsgemäss hergestellten Tetrahydroisochinolin-Derivate der Formel I zu den entsprechenden als wertvolle Zwischenprodukte verwendbaren Decahydroisochinolin-Derivaten der Formel II erfolgt zweckmässigerweise durch Hydrierung in Gegenwart eines für derartige Hydrierungen geeigneten Katalysators, wie Rhodium (zweckmässigerweise 5% Rhodium auf Alox) oder dergleichen und in einem unter den Reaktionsbedingungen inerten organischen Lösungsmittel, wie Ethylacetat oder dergleichen, wobei ein im Rest R allfällig vorhandener Phenylring ebenfalls hydriert wird.

Die Ausgangsstoffe der Formel IV sind bekannt oder nach allgemein bekannten Methoden leicht zugänglich; zudem enthält das nachfolgende Beispiel 1 ausführliche Angaben bezüglich der Herstellung eines solchen Ausgangsstoffes.

In den nachfolgenden Beispielen, welche die vorliegende Erfindung illustrieren, deren Umfang jedoch in keiner Weise einschränken sollen, sind sämtliche Temperaturen in Celsiusgraden angegeben.

### Beispiel 1

A. 1000 g N-Benzyloxycarbonyl-L-phenylalanin wurden in 6400 ml Ethylacetat gelöst. Die Lösung wurde auf -2° (Innentemperatur) gekühlt und unter Argon und unter Rühren innerhalb von 45 Minuten tropfenweise mit 438 g 97%igem N-Ethylmorpholin versetzt, wobei die Innentemperatur in einem Bereich von -1,8° bis -0,6° gehalten wurde. Nach Zugabe von 200 ml Ethylacetat und 10-minütigem Rühren wurden innerhalb von 70 Minuten und bei einer Innentemperatur von -1,8° bis -0,6° tropfenweise 529 g 95%iges Isobutylchloroformat zugegeben, wobei sich ein Niederschlag bildete. Nach Zugabe von 200 ml Ethylacetat wurde die entstandene Suspension 30 Minuten bei -2° Innentemperatur gerührt und dann innerhalb von 80 Minuten tropfenweise mit 353 g t-Butylamin versetzt, wobei die Innentemperatur in einem Bereich von -1,6° bis -0,4° gehalten wurde. Nach Zugabe von 200 ml Ethylacetat wurde 30 Minuten bei -2° Innentemperatur weitergerührt, worauf man die Suspension sich innerhalb von 1 Stunde auf Raumtemperatur erwärmen liess.
   Nach Zugabe von 1400 ml Wasser und 15-minütigem Rühren bei Raumtemperatur erhielt man zwei klare Schichten, und nach 15-minütigem Stehenlassen wurde die gelbliche wässrige Phase (pH 7-8) entfernt. Zur organischen Phase wurden 1000 ml halbgesättigte Natriumbicarbonatlösung gegeben, worauf während 15 Minuten intensiv gerührt wurde. Man liess die Schichten sich während 15 Minuten trennen, entfernte die wässerige Phase, versetzte die organische Phase mit 1000 ml Wasser, rührte 15 Minuten und liess die Schichten sich während 15 Minuten trennen, worauf man die wässerige Phase (pH ≈ 7) entfernte. Die trübe organische Phase wurde unter langsamem Rühren in einem Bad von -15° gekühlt, um die restliche Feuchtigkeit auszufrieren; bei etwa -7° Innentemperatur setzte Kristallisation von Eis ein, und die organische Phase wurde klar; nach 5-minütigem Rühren bei -8° bis -9° Innentemperatur wurde das Eis rasch abfiltriert, und man erhielt eine klare Ethylacetatphase (ca. 9000 ml), welche unter vermindertem Druck eingeengt wurde. Nachdem rund 6000 ml Ethylacetat abdestilliert waren, begann sich ein zähes gelbes Oel auszuscheiden, worauf wieder eine Argonatmosphäre aufgebaut und die Innentemperatur auf 34-40° erhöht wurde.
   Bei einer Innentemperatur von 43-45° wurden tropfenweise 8000 ml Hexan zugegeben, wobei sich eine klare schwach gelbliche Lösung bildete. Diese wurde langsam (5°/Std.) auf -5° abgekühlt, und nach insgesamt 16-stündigem Kühlen wurde der farblose Festkörper bei -5° abgenutscht. Zum Waschen des Filterkuchens wurden 1500 ml Hexan verwendet, womit der Kolben, in welchem die Kristallisation durchgeführt worden war, ausgespült wurde. Nach Trocknen des Filterkuchens in einem Vakuumtrockenschrank bei 40° erhielt man 910 g (77%) N-Benzyloxycarbonyl-L-phenylalanin-t-butylamid vom Schmelzpunkt 99-100°, Reinheit (gemäss HPLC) 99,6%, [α]_{D},²⁰ = 4,9° (c = 1 in Methanol).
   Durch Einengen der Mutterlauge erhielt man ein gelbes Oel, welches aus 100 ml t-Butylmethylether und 500 ml Hexan kristallisiert, abfiltriert und mit 50 ml Hexan/t-Butylmethylether gewaschen wurde; man erhielt weitere 83 g (7,3%) N-Benzyloxycarbonyl-L-phenylalanin-t-butylamid vom Schmelzpunkt 98-100°, Reinheit (gemäss HPLC) 99,0%. Die Gesamtausbeute an N-Benzyloxycarbonyl-L-phenylalanin-t-butylamid betrug 84,3%.
B. 850,8 g N-Benzyloxycarbonyl-L-phenylalanin-t-butylamid wurden bei Raumtemperatur in 3000 ml Toluol gelöst. Zu der trüben Lösung wurde innerhalb von 40 Minuten unter Rühren eine vorbereitete Mischung von 240 ml 95-97%iger Schwefelsäure und 636 ml 99,5%iger Essigsäure zugetropft, worauf innerhalb von 30 Minuten 547 g Dimethoxymethan zugegeben und die stets noch trübe Lösung während 24 Stunden auf 40° (Innentemperatur) erwärmt wurde.
   Nach Zugabe von 1800 ml Toluol wurde das Reaktionsgemisch auf 15° abgekühlt und innerhalb von 50 Minuten bei 15-20° mit 850 ml 25%igem wässerigem Ammoniak versetzt, um das System abzupuffern (auf pH 8-8,5). Um die ausgefallenen Ammoniumsalze aufzulösen, wurden 500 ml Wasser zugegeben, worauf die Mischung bei Raumtemperatur 10 Minuten gerührt und 20 Minuten stehengelassen wurde. Die wässerige Phase wurde entfernt, worauf die organische Phase mit 1000 ml Wasser versetzt und die Mischung bei Raumtemperatur 15 Minuten gerührt und 20 Minuten stehengelassen wurde. Nach Entfernen der wässerigen Phase (pH 7-7,5) wurde die organische Phase unter vermindertem Druck eingeengt. Nachdem rund 4200 ml Toluol abdestilliert waren, verblieb ein zähes Oel, worauf eine Argonatmosphäre aufgebaut und die Innentemperatur auf 45° erhöht wurde.
   Bei einer konstanten Aussentemperatur von 70° wurden 2000 ml Hexan so zugegeben, dass die Innentemperatur stets 50-55° betrug. Die entstehende klare gelbe Lösung wurde dann nicht zu schnell (20°/Std.) auf -10° abgekühlt und 1 Stunde bei -10° gerührt, worauf die ausgefallenen Kristalle abgenutscht wurden. Um im Kolben verbliebenes Material zu entfernen, wurden 1000 ml Hexan zugegeben und bei -10° heftig gerührt; die kalte Suspension wurde verwendet, um den Filterkuchen zu waschen. Man erhielt 578 g (66%) Benzyl 3(S)-(t-butylcarbamoyl)-1,2,3,4-tetrahydro-3-isochinolincarboxylat in Form weisslicher Kristalle vom Schmelzpunkt 105-106°, Reinheit (gemäss HPLC) 99,0%, [α]_{D},²⁰ = -34,2° (c = 1 in Methanol).

### Beispiel 2

5,7 kg Benzyl 3(S)-(t-butylcarbamoyl)-1,2,3,4-tetrahydro-3-isochinolincarboxylat wurden in 40 l Ethanol gelöst und in Gegenwart von 1,1 kg 10%iger Palladiumkohle bei einem Druck von 10 bar und einer Innentemperatur von 20-25° in einem Autoklaven hydriert. Nach rund 2 Stunden kam die Wasserstoffaufnahme zum Stillstand, worauf man den Inhalt des Autoklaven filtrierte, den Autoklaven und den Filterrückstand zweimal mit je 20 l Ethanol nachspülte und die vereinigten Filtrate eindampfte, wobei rund 3,6 kg rohes N-t-Butyl-1,2,3,4-tetrahydro-3(S)-isochinolincarboxamid als beiges Kristallisat anfielen.

Zu diesem Rohprodukt gab man 40 l Hexan, rührte, erhitzte auf rund 65° (Innentemperatur), filtrierte und wusch mit zweimal je 15 l heissem (60°) Hexan nach. Anschliessend wurden rund 35 l Hexan abdestilliert, worauf man über Nacht auf Raumtemperatur abkühlen liess, die ausgefallenen Kristalle abnutschte und diese portionenweise mit insgesamt 10 l Hexan und 10 l Pentan wusch. Nach Trocknen im Vakuum bei 40° erhielt man 2,9 kg (79%) N-t-Butyl-1,2,3,4-tetrahydro-3(S)-isochinolincarboxamid in Form weisser Kristalle vom Schmelzpunkt 95-96°, Reinheit (gemäss GC) 99,6%, [α]₅₈₉,²⁰ = -109,5° (c = 1 in Methanol).

### Beispiel 3

15 kg N-t-Butyl-1,2,3,4-tetrahydro-3(S)-isochinolincarboxamid wurden in 100 l Ethylacetat suspendiert und in Gegenwart von 3 kg Rhodium/Alox (5%) unter Rühren bei einem Druck von 80 bar und bei einer Innentemperatur von 80° hydriert. Nach rund 4 Stunden kam die Wasserstoffaufnahme zum Stillstand, worauf man das Reaktionsgemisch filtrierte, den Filterrückstand mit 40 l Ethylacetat nachwusch und die vereinigten Filtrate im Vakuum zur Trockene eindampfte. Nach Aufnehmen des Rückstandes in 40 l Hexan, Verrühren und Eindampfen erhielt man 15,3 kg rohes N-t-Butyl-decahydro-(4aS,8aS)-isochinolin-3(S)-carboxamid in Form eines hellen Kristallisats.

Zu diesem Rohprodukt gab man 45 l Hexan, rührte, erhitzte auf 65° (Innentemperatur), filtrierte und wusch erst mit 20 l und dann mit 10 l heissem (60°) Hexan nach. Anschliessend wurden rund 30 l Hexan abdestilliert, worauf man über Nacht auf 0° abkühlte, die ausgefallenen Kristalle abzentrifugierte und diese mit 10 l kaltem (0°) Hexan nachwusch.

Das erhaltene feuchte Produkt (12 kg) wurde mit 110 l Hexan versetzt, worauf man rührte, auf 65° (Innentemperatur) erhitzte, filtrierte und dann über Nacht auf 0° abkühlte. Die ausgefallenen Kristalle wurden abzentrifugiert, mit 10 l kaltem (0°) Hexan nachgewaschen und bei 40° im Vakuum getrocknet. Man erhielt 9,1 kg (59%) N-t-Butyl-decahydro-(4aS,8aS)-isochinolin-3(S)-carboxamid in Form weisser Kristalle vom Schmelzpunkt 112-114°, Reinheit (gemäss GC) nahezu 100%, [α]₅₈₉,²⁰ = -73 bis -74,2° (c = 0,5 in Methanol).

## Patentansprüche

1. Verfahren zur Herstellung von 1,2,3,4-Tetrahydro-3-isochinolin-Derivaten der allgemeinen Formel 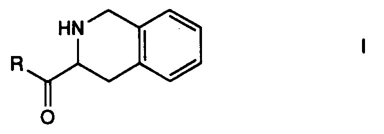 worin R Hydroxy, nieder Alkylamino, nieder Alkoxy oder Phenyl-C₂₋₆-Alkoxy, welches am Phenylring durch nieder Alkyl oder
nieder Alkoxy ein- oder mehrfach substituiert sein kann, bedeutet, dadurch gekennzeichnet, dass man ein Phenethylamin-Derivat der allgemeinen Formel 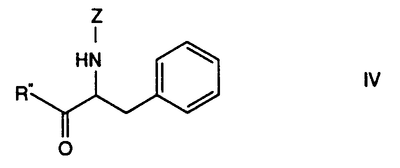 worin Z Benzyloxycarbonyl und R'' Hydroxy, nieder Alkylamino, nieder Alkoxy oder Phenyl-nieder Alkoxy, welches am Phenylring durch nieder Alkyl oder nieder Alkoxy ein- oder mehrfach substituiert sein kann, bedeuten,
bei einer Temperatur zwischen etwa Raumtemperatur und etwa 50°C in Gegenwart von Schwefelsäure in Essigsäure mit Formaldehyd umsetzt und in dem erhaltenen Cyclisationsprodukt der allgemeinen Formel 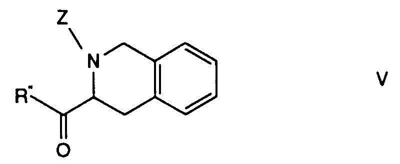 worin R'' und Z obige Bedeutung besitzen, die Benzyloxycarbonylgruppe (Z) abspaltet.

2. Verfahren nach Anspruch 1, worin R nieder Alkylamino bedeutet.

3. Verfahren nach Anspruch 1 oder 2, dadurch gekennzeichnet, dass man den Formaldehyd in Form von wässeriger Formaldehydlösung oder in Form von Paraformaldehyd oder in Form eines Di-nieder-Alkylacetals verwendet.

4. Verfahren nach Anspruch 3, dadurch gekennzeichnet, dass man Dimethoxymethan verwendet.

5. Verfahren nach einem der Ansprüche 1-4, dadurch gekennzeichnet, dass man als Lösungsmittel Toluol oder einen Ueberschuss an Essigsäure verwendet.

6. Verfahren nach Anspruch 5, dadurch gekennzeichnet, dass man als Lösungsmittel Toluol verwendet.

7. Verfahren nach einem der Ansprüche 1-6, dadurch gekennzeichnet, dass das Phenethylamin-Derivat der Formel IV in Toluol mit 3 Mol-Aequivalenten Dimethoxymethan und 2 Mol-Aequivalenten 7,2M Schwefelsäure in Essigsäure 24 bis 25 Stunden bei etwa 40°C zur Reaktion gebracht wird.

8. Verfahren nach einem der Ansprüche 1-7, dadurch gekennzeichnet, dass man die Benzyloxycarbonylgruppe (Z) aus dem Cyclisationsprodukt der Formel V hydrogenolytisch abspaltet.

9. Verfahren nach Anspruch 8, dadurch gekennzeichnet, dass man das Cyclisationsprodukt der Formel V in Gegenwart eines Palladiumkatalysators hydriert.

## Claims

1. A process for the manufacture of 1,2,3,4-tetrahydro-3-isoquinoline derivatives of the general formula 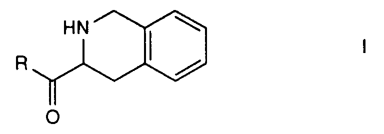 wherein R signifies hydroxy, lower alkylamino, lower alkoxy or phenyl-C₂₋₆-alkoxy which can be substituted by one or more lower alkyl or lower alkoxy substituents on the phenyl ring,
characterized by reacting a phenethylamine derivative of the general formula 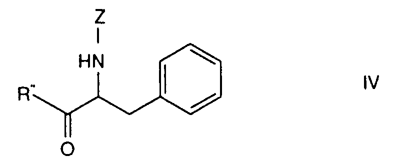 wherein Z signifies benzyloxycarbonyl and R'' signifies hydroxy, lower alkylamino, lower alkoxy or phenyl-lower-alkoxy which can be substituted by one or more lower alkyl or lower alkoxy substituents on the phenyl ring,
with formaldehyde in the presence of sulphuric acid in acetic acid at a temperature between about room temperature and about 50°C and cleaving off the benzyloxycarbonyl group (Z) in the resulting cyclization product of the general formula 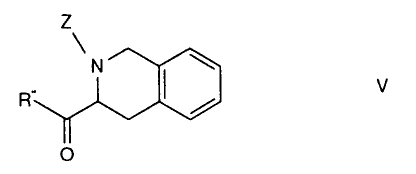 wherein R'' and Z have the above significance.

2. A process according to claim 1, wherein R signifies lower alkylamino.

3. A process according to claim 1 or 2, characterized in that the formaldehyde is used in the form of aqueous formaldehyde solution or in the form of paraformaldehyde or in the form of a di-lower-alkyl acetal.

4. A process according to claim 3, characterized in that dimethoxymethane is used.

5. A process according to any one of claims 1-4, characterized in that toluene or an excess of acetic acid is used as the solvent.

6. A process according to claim 5, characterized in that toluene is used as the solvent.

7. A process according to any one of claims 1-5, characterized in that the phenethylamine derivative of formula IV is reacted in toluene with 3 mol equivalents of dimethoxymethane and 2 mol equivalents of 7.2M sulphuric acid in acetic acid for 24 to 25 hours at about 40°C.

8. A process according to any one of claims 1-7, characterized in that the benzyloxycarbonyl group (Z) is cleaved off hydrogenolytically from the cyclization product of formula V.

9. A process according to claim 8, characterized in that the cyclization product of formula V is hydrogenated in the presence of a palladium catalyst.

## Revendications

1. Procédé de préparation de dérivés de 1,2,3,4-tétrahydro-isoquinoléines substituées en 3, de formule générale 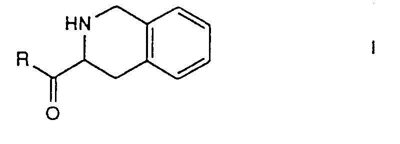 dans laquelle R représente un hydroxy, alkyle inférieur-amino, alcoxy inférieur ou phényl-alcoxy en C₂ à C₆ , qui peut être mono- ou polysubstitué sur le noyau phényle par un alkyle inférieur ou un alcoxy inférieur, caractérisé en ce qu'on fait réagir avec du formaldéhyde un dérivé de phénéthylamine de formule générale 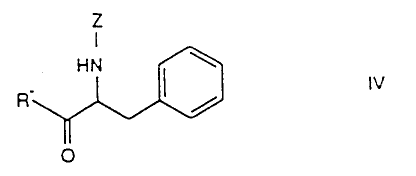 dans laquelle Z représente un benzyloxycarbonyle et R'' un hydroxy, un alkyle inférieur-amino, un alcoxy inférieur ou un phényl-alcoxy inférieur qui peut être mono- ou polysubstitué sur le noyau phényle par un alkyle inférieur ou un alcoxy inférieur,
à une température comprise entre les environs de la température ambiante et environ 50°C en présence d'acide sulfurique dans l'acide acétique, et en ce qu'on sépare le groupe benzyloxycarbonyle (Z) dans le produit de cyclisation obtenu, de formule générale 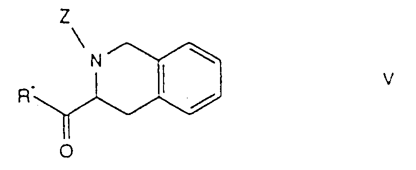 dans laquelle R'' et Z ont la signification donnée ci-dessus.

2. Procédé selon la revendication 1, dans lequel R représente un alkyle inférieur-amino.

3. Procédé selon la revendication 1 ou 2, caractérisé en ce qu'on utilise le formaldéhyde sous la forme d'une solution aqueuse de formaldéhyde ou sous forme de paraformaldéhyde , ou sous la forme d'un di-alkyle inférieur-acétal.

4. Procédé selon la revendication 3, caractérisé en ce qu'on utilise le diméthoxyméthane.

5. Procédé selon l'une des revendications 1-4, caractérisé en ce qu'on utilise comme solvant le toluène ou un excès d'acide acétique.

6. Procédé selon la revendication 5, caractérisé en ce qu'on utilise comme solvant le toluène.

7. Procédé selon l'une des revendications 1-6, caractérisé en ce qu'on fait réagir pendant 24 à 25 heures à environ 40°C le dérivé de phénéthylamine de formule IV dans le toluène avec 3 équivalents molaires de diméthoxyméthane et 2 équivalents molaires d'acide sulfurique 7,2 M dans l'acide acétique.

8. Procédé selon l'une des revendications 1-7, caractérisé en ce qu'on sépare par hydrogénolyse le groupe benzyloxycarbonyle (Z) du produit de cyclisation de formule V.

9. Procédé selon la revendication 8, caractérisé en ce qu'on hydrogène le produit de cyclisation de formule V en présence d'un catalyseur de palladium.
